# EUROPÄISCHE PATENTSCHRIFT

(11) **EP 1 175 200 B1**
(45) Veröffentlichungstag und Bekanntmachung des Hinweises auf die Patenterteilung: **24.11.2010**
(21) Anmeldenummer: 00929411.7
(22) Anmeldetag: 22.04.2000
(51) Int. Cl.: A61K 8/02

(54) **VERWENDUNG NANOSKALIGER ANTIMIKROBIELLER WIRKSTOFFE IN KÖRPERDEODORANTIEN**
USE OF NANOSCALAR ANTIMICROBIAL ACTIVE INGREDIENTS IN BODY DEODORANTS
UTILISATION DANS DES DEODORANTS CORPORELS DE PRINCIPES ACTIFS ANTIMICROBIENS NANOMETRIQUES

(30) Priorität: 30.04.1999 DE 19919769
(43) Veröffentlichungstag der Anmeldung: 30.01.2002
(73) Patentinhaber: Cognis IP Management GmbH, 40589 Düsseldorf (DE)
(72) Erfinder: SCHRÖDER, Christine, D-40593 Düsseldorf (DE); ROTH, Marcel, D-40591 Düsseldorf (DE); BANOWSKI, Bernhard, D-40597 Düsseldorf (DE); LEINEN, Hans, Theo, D-40229 Düsseldorf (DE); GLASL, Johann, D-42719 Solingen (DE)
(86) Internationale Anmeldenummer: PCT/EP2000/003659
(87) Internationale Veröffentlichungsnummer: WO 2000/066074

(56) Entgegenhaltungen:
- EP-A- 0 200 548
- EP-A- 0 423 002
- WO-A-90/10635
- DATABASE WPI Week 199814 Derwent Publications Ltd., London, GB; AN 1998-148305 XP002146389 & JP 09 299460 A (SHOKUBAI KASEI KOGYO), 25. November 1997 (1997-11-25)
- DATABASE EPODOC JP9299460 A (CATALYSTS & CHEM IND CO) 25. November 1997 (1997-11-25)

## Beschreibung

Die Erfindung betrifft die Verwendung von antimikrobiellen Wirkstoffen in nanoskaliger Form zur Herstellung von Körperdeodorantien.

Körperdeodorantien, auch Desodorantien genannt, sind Mittel, die Körpergerüchen entgegenwirken, sie überdecken oder beseitigen. Körpergerüche entstehen durch die Einwirkung von Hautbakterien auf apokrinen Schweiß, wobei unangenehm riechende Abbauprodukte gebildet werden. Dementsprechend enthalten Deodorantien Wirkstoffe, die als antibakterielle Mittel, Enzyminhibitoren, Geruchsabsorber oder Geruchsüberdecker fungieren.

Unter nanoskaligen Stoffen sind Stoffe zu verstehen, deren Teilchendurchmesser in der Richtung der größten Ausdehnung der Teilchen weniger als 1000 nm (Nanometer) beträgt. In der vorliegenden Schrift wird synonym zu dem Begriff "nanoskalig" auch der Begriff "nanopartikulär" benutzt. Nanoskalige Wirkstoffe werden in der Literatur insbesondere als Mittel beschrieben, um eine kontrollierte Freisetzung des Wirkstoffs über einen längeren Zeitraum zu erreichen. So sind beispielsweise aus der WO 98/14174 Nanopartikel zur parenteralen therapeutischen Verwendung bekannt, welche aus einem pharmakologisch aktiven Stoff eingekapselt in eine Hülle aus einem biologisch abbaubaren Polymer bestehen. Als Beispiele für pharmakologisch aktive Stoffe sind u. a. antibakterielle Stoffe wie Chloramphenicol und Vancomycin sowie antimikrobielle Stoffe wie Penicilline und Cephalosporine genannt. Antimikrobielle Produkte enthaltend nanoskalige Schiff'sche Basen von aromatischen Aldehyden sind bekannt aus der DE 4402103, welche die Verwendung dieser Produkte zur langanhaltenden antimikrobiellen Ausrüstung von Textilien beschreibt. Die Anmeldung CA 2,111,523 beschreibt Desinfektionsmittel, die neben anderen Bestandteilen auch oberflächenmodifizierte nanopartikuläre antimikrobielle Wirkstoffe enthalten. Als Beispiel wird eine desinfizierende Reiniger-Formulierung angegeben. Die Anmeldung CA 2,111,522 beschreibt Zusammensetzungen mit langanhaltender keimtötender Wirkung, welche oberflächenmodifizierte nanopartikuläre antimikrobielle Wirkstoffe enthalten. Als Anwendungen dieser Zusammensetzungen werden Desinfektionsmittel zur Oberflächenbehandlung beschrieben, die dauerhafte antimikrobiell wirksame Filme auf der behandelten Oberfläche liefern.

WO 00/35411 beschreibt ein Verfahren zur Herstellung wäßriger Gele aus feinteiligen Oxiden, Oxidhydraten und Hydroxiden von Calcium, Magnesium, Aluminium, Titan, Zirkon oder Zink und die Verwendung dieser Gele zur Körperdeododierung.

Der Stand der Technik liefert jedoch keine Hinweise darauf, daß nanopartikuläre antimikrobielle Stoffe vorteilhaft als Wirkstoffe im Bereich der Körperdeodorantien eingesetzt werden können. Einerseits ist dem Fachmann zwar bekannt, daß antimikrobielle Wirkstoffe sowohl z. B. im Bereich der Oberflächendesinfektion als auch im Bereich der Körperdeodorantien Einsatz finden. Andererseits ist ihm jedoch ebenso bekannt, daß die Art der Anwendung sowie die Anforderungen an die Wirkstärke, das Wirkspektrum und die Formulierung der Wirkstoffe in den unterschiedlichen Einsatzgebieten so verschieden sind, daß die in dem einen Anwendungsbereich gewonnenen Erkenntnisse nicht in naheliegender Weise auf einen anderen Anwendungsbereich übertragen werden können.

Bei den in Körperdeodorantien verwendeten antimikrobiellen Wirkstoffen tritt bedingt durch die physikochemischen Eigenschaften bzw. den Eigengeruch dieser Wirkstoffe in der Praxis häufig das Problem auf, daß sie in Formulierungen der Deodorantien nur schwer oder nur in unzureichenden Konzentrationen einarbeitbar sind und die erhaltenen Formulierungen eine unbefriedigende antimikrobielle Wirkung zeigen.

Darüber hinaus besteht beim Verbraucher ein Bedürfnis nach Körperdeodorantien, die ohne Einbußen bei der deodorierenden Wirkung mit verringerten Einsatzkonzentrationen des Wirkstoffs auskommen und damit gesundheitliche, ökonomische und / oder ökologische Vorteile bieten.

Eine Aufgabe der Erfindung bestand somit darin, die Herstellung von Körperdeodorantien unter Verwendung solcher antimikrobieller Wirkstoffe zu ermöglichen, die z. B. aufgrund ihrer schlechten Löslichkeit oder ihres starken Eigengeruchs auf herkömmlichen Wegen nur schwierig bzw. nicht in ausreichenden Konzentrationen in Körperdeodorantien eingearbeitet werden können.

Eine weitere Aufgabe der Erfindung bestand darin, Körperdeodorantien mit einer für die Anwendung ausreichenden antimikrobiellen Wirksamkeit und gleichzeitig verringertem Gehalt an antimikrobiellen Wirkstoffen bereitzustellen.

Die Aufgabe wurde dadurch gelöst, daß die antimikrobiellen Wirkstoffe ausgenommen feinteilige Oxide, Oxidhydrate oder Hydroxide des Calciums, Magnesiums, Aluminiums, Titans, Zirkoniums oder des Zink in Form von Nanopartikeln mit einem Teilchendurchmesser im Bereich von 5 bis 500 nm, vorzugsweise von 10 bis 150 nm, zur Herstellung der Körperdeodorantien verwendet werden.

Ein Gegenstand der Erfindung ist daher die Verwendung von nanoskaligen antimikrobiellen Wirkstoffen ausgenommen feinteilige Oxide, Oxidhydrate oder Hydroxide des Calciums, Magnesiums, Aluminiums, Titans, Zirkoniums oder des Zink mit einem Teilchendurchmesser im Bereich von 5 bis 500 nm, bevorzugt von 10 bis 150 nm, zur Herstellung von Körperdeodorantien, insbesondere von deodorierenden Aerosolen, Pumpsprays, Roll-ons und Stiften. Die Verwendung der nanoskaligen antimikrobiellen Wirkstoffe eignet sich besonders zur Herstellung von Produkten, bei welchen keine bakterizide, sondern lediglich eine bakteriostatische Wirkung gewünscht ist.

Überraschenderweise wurde gefunden, daß damit beispielsweise folgende Vorteile verbunden sind:
a) die Einarbeitbarkeit von antimikrobiellen Wirkstoffen in Formulierungen für Deodorantien wird dergestalt verbessert, daß lipophile Wirkstoffe leichter in wässrige Formulierungen und hydrophile Wirkstoffe leichter in nichtwäßrige bzw. wasserarme Formulierungen eingearbeitet werden können
b) die Wirksamkeit der Wirkstoffe aus den Formulierungen wird erhöht. Dies bedeutet, daß bei gewichtsgleichem Einsatz der nanopartikuläre Wirkstoff gegenüber dem gleichen Wirkstoff in höherer, dem Stand der Technik entsprechender Partikelgröße eine stärkere antimikrobielle Wirkung hervorruft.
c) im Falle von Wirkstoffen mit störendem Eigengeruch kann im Fall einer Oberflächenmodifizierung der nanoskaligen Partikel der Geruch abgeschwächt oder sogar unterdrückt werden.

Für die erfindungsgemäße Verwendung eignen sich besonders antibakterielle Wirkstoffe mit einer weitgehend selektiven Wirksamkeit gegenüber Bakterien, welche an der Entstehung geruchsbildender Stoffe im Körperschweiß beteiligt sind. Beim Einsatz antimikrobieller Wirkstoffe ist zu beachten, daß die Population der betroffenen Bakterien lediglich kontrolliert und ein zu starkes Wachstum verhindert werden soll (bakteriostatische Wirkung), diese jedoch nicht vollständig abgetötet werden soll (was einer bakterizide Wirkung entsprechen würde).

Als erfindungsgemäße antimikrobielle Wirkstoffe sind bevorzugt alle gegen grampositive Bakterien wirksamen Stoffe geeignet.. Besonders bevorzugt sind Wirkstoffe, die gegen Corynebacterium xerosis wirksam sind.
Zu den erfindungsgemäßen Wirkstoffen zählen beispielsweise
- 4-Hydroxybenzoesäure, ihre Salze mit Alkali- oder Erdalkalimetallen oder ihre Ester mit linearen oder verzweigten Alkoholen mit 1-10 C-Atomen
- N-(4-Chlorphenyl)-N'-(3,4-dichlor-phenyl)-harnstoff
- 2,4,4'-Trichlor-2'-hydroxy-diphenylether (Triclosan)
- 4-Chlor-3,5-dimethylphenol
- 2,2'-Methylen-bis(6-brom-4-chlorphenol)
- 3-Methyl-4-(1-methylethyl)phenol
- 2-Benzyl-4-chlorphenol
- 3-(4-Chlorphenoxy)-1,2-propandiol
- 3-lod-2-propinyl-butylcarbamat
- Chlorhexidin
- 3,4,4'-Trichlorcarbanilid (TTC)
- 1-Hydroxy-4-methyl-6-(2,4,4-trimethylpentyl)-2(1H)-pyridinon, Ethanolaminsalz (1:1) (Octopirox)
- antimikrobielle Riechstoffe wie z. B. Thymol oder Menthol
- Glycerinmonolaurat (GML)
- Diglycerinmonocaprinat (DMC)
- Zinksalze wie z. B. Zinkglycinat, Zinklactat oder Zinkphenolsulfonat
- Phytosphingosine
- 1,2-Dodecandiol
- Undecylensäure, ihre Salze mit Alkali- oder Erdalkalimetallen oder ihre Ester mit linearen oder verzweigten Alkoholen mit 1-10 C-Atomen
- Salicylsäure-N-alkylamide, wobei die Alkylreste 1-22 C-Atome enthalten und linear oder verzweigt sein können
   und deren Gemische.

Besonders bevorzugt als erfindungsgemäße antimikrobielle Wirkstoffe sind Salicylsäure-N-octylamid und/oder Salicylsäure-N-decylamid, 2,4,4'-Trichlor-2'-hydroxydiphenylether sowie antimikrobiell wirksame Riechstoffe.

Die erfindungsgemäßen nanoskaligen Wirkstoffe bestehen aus einer diskreten Phase des Wirkstoffs, an dessen Oberfläche vorzugsweise mindestens ein oberflächenmodifizierender Stoff adsorbiert ist. Als oberflächenmodifizierende Stoffe besonders geeignet sind Emulgatoren und/oder Schutzkolloide. Die Ummantelung der Partikel mit Emulgatoren und/oder Schutzkolloiden führt dazu, daß eine nachträgliche Agglomeration der Partikel nicht stattfindet.

Als Emulgatoren kommen beispielsweise nichtionogene Tenside aus mindestens einer der folgenden Gruppen in Frage:
(1) Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
(2) C_{12/18}-Fettsäuremono- und -diester von Anlagerungsprodukten von 1 bis 30 Mol Ethylenoxid an Glycerin;
(3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
(4) Alkylmono- und -oligoglycoside mit 8 bis 22 Kohlenstoffatomen im Alkylrest und deren ethoxylierte Analoga;
(5) Anlagerungsprodukte von 15 bis 60 Mol Ethylenoxid an Ricinusöl und/oder gehärtetes Ricinusöl;
(6) Polyol- und insbesondere Polyglycerinester, wie z.B. Polyglycerinpolyricinoleat, Polyglycerinpoly-12-hydroxystearat oder Polyglycerindimerat. Ebenfalls geeignet sind Gemische von Verbindungen aus mehreren dieser Substanzklassen;
(7) Anlagerungsprodukte von 2 bis 15 Mol Ethylenoxid an Ricinusöl und/oder gehärtetes Ricinusöl;
(8) Partialester auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipenta-erythrit, Zuckeralkoholen (z.B. Sorbit), Sucrose, AIkylglucosiden (z.B. Methylglucosid, Butylglucosid, Lauryl-glucosid) sowie Polyglucosiden (z.B. Cellulose);
(9) Mono-, Di- und Trialkylphosphate sowie Mono-, Di- und/oder Tri-PEGalkylphosphate und deren Salze;
(10) Wollwachsalkohole;
(11) Polysiloxan-Polyalkyl-Polyether-Copolymere bzw. entsprechende Derivate;
(12) Mischester aus Pentaerythrit, Fettsäuren, Citronensäure und Fettalkohol gemäß DE-PS 1165574 und/oder Mischester von Fettsäuren mit 6 bis 22 Kohlenstoffatomen, Methylglucose und Polyolen, vorzugsweise Glycerin oder Polyglycerin sowie
(13) Polyalkylenglycole.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologen-gemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus DE-PS 2024051 als Rückfettungsmittel für kosmetische Zubereitungen bekannt.

C_{8/18}-Alkylmono- und -oligoglycoside, ihre Herstellung und ihre Verwendung sind aus dem Stand der Technik bekannt. Ihre Herstellung erfolgt insbesondere durch Umsetzung von Glucose oder Oligosac-chariden mit primären Alkoholen mit 8 bis 18 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Typische Beispiele für anionische Emulgatoren sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Hydroxymischethersulfate, Monoglycerid(ether)sulfate, Fettsäureamid(ether)sulfate, Mono- und Dialkyl-sulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren wie beispielsweise Acyllactylate, Acyl-tartrate, Acylglutamate und Acylaspartate, Alkyloligoglucosidsulfate, Proteinfettsäurekondensate (insbe-sondere pflanzliche Produkte auf Weizenbasis) und Alkyl(ether)phosphate. Sofern die anionischen Ten-side Polyglycoletherketten enthalten, können diese eine konventionelle, vorzugsweise jedoch eine ein-geengte Homologenverteilung aufweisen.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylamino-propyl-N,N-dimethylammoniumglycinate, beispielsweise das Kokosacylaminopropyldimethyl-ammonium-glycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung Cocamidopropyl Betaine bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampho-lytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Neben den ampholytischen kommen auch quartäre Emulgatoren in Betracht, wobei solche vom Typ der Esterquats, vorzugsweise methyl-quaternierte Difettsäuretriethanolaminester-Salze, besonders bevorzugt sind. Typische Beispiele für anionische Emulgatoren sind Alkylsulfate, Alkylethersulfate und Monoglycerid(ether)sulfate.

In der Regel werden die Wirkstoffe und die Emulgatoren im Gewichtsverhältnis 1 : 100 bis 100 : 1, vorzugsweise 1 : 25 bis 25 : 1 und insbesondere 1 : 10 bis 10 : 1 eingesetzt. Besonders bevorzugt sind solche Emulgatoren, welche zur Ausbildung von Mikroemulsionen befähigt sind.

Geeignete Schutzkolloide sind z.B. Gelatine, Casein, Gummi arabicum, Lysalbinsäure, Stärke, Carboxymethylcellulose oder modifizierte Carboxymethylcellulose sowie Polymere, wie etwa Polyvinylalkohole, Polyvinylpyrrolidone, Polyalkylenglycole und Polyacrylate.

Ein weiterer Erfindungsgegenstand ist somit die erfindungsgemäße Verwendung nanoskaliger antimikrobieller Wirkstoffe, bei welchen die Nanopartikel von einem oder mehreren Emulgatoren und / oder Schutzkolloiden ummantelt vorliegen.

Die erfindungsgemäßen Nanopartikel können beispielsweise hergestellt werden, indem man
(a) Wirkstoffe in eine flüssige Phase, in der sie nicht löslich sind, einbringt,
(b) die resultierende Mischung über den Schmelzpunkt der Wirkstoffe erwärmt,
(c) der resultierenden Ölphase eine wirksame Menge mindestens eines Emulgators zusetzt und schließlich
(d) die Emulsion unter den Schmelzpunkt der Wirkstoffe abkühlt.
Gegenstand der Erfindung ist daher auch die erfindungsgemäße Verwendung nanoskaliger antimikrobieller Wirkstoffe, die nach diesem Verfahren hergestellt sind.

Ein weiteres Verfahren zur Herstellung von Nanopartikeln durch rasche Entspannung von überkritischen Lösungen (Rapid Expansion of Supercritical Solutions RESS) ist beispielsweise aus dem Aufsatz von S.Chihlar, M.Türk und K.Schaber in Proceedings World Congress on Particle Technology 3, Brighton, 1998 bekannt. Um zu verhindern, daß die Nanopartikel wieder zusammenbacken, empfiehlt es sich, die Ausgangsstoffe in Gegenwart geeigneter Schutzkolloide oder Emulgatoren zu lösen und/oder die kritischen Lösungen in wäßrige und/oder alkoholische Lösungen der Schutzkolloide bzw. Emulgatoren oder aber in kosmetische Öle zu entspannen, welche ihrerseits wieder gelöste Emulgatoren und/oder Schutzkolloide enthalten können.

Ein weiteres geeignetes Verfahren zur Herstellung der nanoskaligen Partikel bietet die Evaporations-technik. Hierbei werden die Ausgangsstoffe zunächst in einem geeigneten organischen Lösungsmittel (z.B. Alkane, pflanzliche Öle, Ether, Ester, Ketone, Acetale und dergleichen) gelöst. Anschließend werden die Lösungen derart in Wasser oder einem anderen Nicht-Lösungsmittel, in der Regel in Ge-genwart einer darin gelösten oberflächenaktiven Verbindung gegeben, daß es durch die Homogenisierung der beiden nicht miteinander mischbaren Lösungsmittel zu einer Ausfällung der Nanopartikel kommt, wobei das organische Lösungsmittel vorzugsweise verdampft. Anstelle einer wäßrigen Lösung können auch O/W-Emulsionen bzw. O/W-Mikroemulsionen eingesetzt werden. Als oberflächenaktive Verbindungen können die bereits eingangs erläuterten Emulgatoren und Schutzkolloide verwendet werden. Eine weitere Möglichkeit zur Herstellung von Nanopartikeln besteht in dem sogenannten GAS-Verfahren (Gas Anti Solvent Recrystallization). Das Verfahren nutzt ein hochkomprimiertes Gas oder überkritisches Fluid (z.B. Kohlendioxid) als Nicht-Lösungsmittel zur Kristallisation von gelösten Stoffen. Die verdichtete Gasphase wird in die Primärlösung der Ausgangsstoffe eingeleitet und dort absorbiert, wodurch sich das Flüssigkeitsvolumen vergrößert, die Löslichkeit abnimmt und feinteilige Partikel aus-geschieden werden.
Ähnlich geeignet ist das PCA-Verfahren (Precipitation with a Compressed Fluid Anti-Solvent). Hier wird die Primärlösung der Ausgangsstoffe in ein überkritisches Fluid eingeleitet, wobei sich feinstverteilte Tröpfchen bilden, in denen Diffusionsvorgänge ablaufen, so daß eine Ausfällung feinster Partikel erfolgt. Beim PGSS-Verfahren (Particles from Gas Saturated Solutions) werden die Ausgangsstoffe durch Aufpressen von Gas (z.B. Kohlendioxid oder Propan) aufgeschmolzen. Druck und Temperatur erreichen nahe- oder überkritische Bedingungen. Die Gasphase löst sich im Feststoff und bewirkt eine Absenkung der Schmelztemperatur, der Viskosität und der Oberflächenspannung. Bei der Expansion durch eine Düse kommt es durch Abkühlungseffekte zur Bildung feinster Teilchen.

Die aufgeführten Herstellverfahren für die erfindungsgemäßen Nanopartikel sind lediglich beispielhaft zu verstehen und stellen keine Einschränkung dar.

Die unter der erfindungsgemäßen Verwendung der nanoskaligen antimikrobiellen Wirkstoffe erhältlichen Körperdeodorantien können ferner als weitere Hilfs- und Zusatzstoffe beispielsweise Fettsäuren in Form ihrer Alkaliseifen, Polyole, niedere Alkohole, Enzyminhibitoren, Geruchsabsorber, Geruchsüberdecker, Wasser, Komplexbildner, Antioxidantien, Konservierungsmittel, Duftstoffe, Färbemittel, Trübungsmittel, Perlglanzpigmente, feinteilige Kieselsäure, Konsistenzgeber, Gelbildner, Wachse, Fettalkohole, Emulgatoren, Verdicker sowie weitere geeignete Rezepturgrundlagen enthalten.
Unter Fettsäuren sind Carbonsäuren mit 16 - 22 C-Atomen zu verstehen wie z. B. Palmitinsäure, Stearinsäure und Behensäure oder technische Gemische, die überwiegend aus solchen Fettsäuren bestehen, z. B. gehärtete Palmölfettsäure oder gehärtete Talgfettsäure.
Unter Polyolen sind solche mit 3 - 6 C-Atomen und 2-6 Hydroxylgruppen zu verstehen wie z. B. Ethylenglycol, 1,2-Propylenglycol, 1,3-Propylenglycol, 1,2-Butylenglycol, 1,3-Butylenglycol, 1,4-Butylenglycol, Glycerin, Erythrit, Pentaerythrit, Trimethylolpropan, Sorbit, Anhydrosorbit, Cyclohexantriol oder Inosit.

Als niedere Alkohole können die Zubereitungen z. B. Ethanol oder Isopropanol enthalten.

Als Enzyminhibitoren sind beispielsweise Esteraseinhibitoren geeignet. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® CAT, Henkel KGaA, Düsseldorf/FRG). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbnonsäuren und deren Ester wie beispielsweise Citronensäure, Äpfelsäure, Weinsäure oder Weinsäurediethylester, sowie Zinkglycinat.

Als Geruchsabsorber eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, daß dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfümöle, die zusätzlich zu ihrer Funktion als Geruchsüberdecker den Deodorantien ihre jeweilige Duftnote verleihen. Als Parfümöle seien beispielsweise genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, p-tert.-Butylcyclohexylacetat, Linalylacetat, Phenylethylacetat, Linalylbenzoat, Benzylformiat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilat, Irotyl und Floramat allein oder in Mischungen, eingesetzt.

Um die Wirkstoffe auf eine dosierbare, sparsame, bequeme und kosmetisch ansprechende Weise auf die Haut applizieren zu können, sind sie in geeignete Rezepturgrundlagen einzuarbeiten. Als wichtigste Rezepturgrundlagen sind zu nennen: alkoholische und wäßrig/alkoholische Lösungen, Emulsionen, Gele, Stifte - z. B. glykolische Seifenstifte - Öle, Wachs/Fett-Massen und Puder. Als zusätzliche Hilfsstoffe können z. B. Stabilisatoren, Konsistenzgeber und Schauminhibitoren zum Einsatz kommen.

Als Anbietungsformen für Deodorantien eignen sich Aerosole, Pumpsprays, Roll-ons, Stifte und Gele, darüber hinaus auch Cremes und Puder.

Die Einsatzmenge der nanoskaligen Verbindungen wird so gewählt, daß die Konzentration der in den Nanopartikeln enthaltenen antimikrobiellen Wirkstoffe üblicherweise in der Größenordnung von 0,01 bis 5, vorzugsweise 0,1 bis 2 Gew.-% bezogen auf die Zubereitungen liegt.

Zur Herstellung der erfindungsgemäßen Mund- und / oder Zahnpflegemittel werden die nanoskaligen antimikrobiellen Wirkstoffe in dem Fachmann bekannter Weise mit den übrigen Rezepturbestandteilen vermengt.

Ein weiterer Gegenstand der Erfindung sind antimikrobielle Wirkstoffe enthaltende Körperdeodorantien, die dadurch gekennzeichnet sind, daß der antimikrobielle Wirkstoff in Form von Nanopartikeln mit einem Teilchendurchmesser im Bereich von 5 bis 500 nm, vorzugsweise von 10 bis 150 nm, eingearbeitet ist.
Weitere Ausgestaltungen und / oder Weiterentwicklungen ergeben sich aus den Unteransprüchen.

### Beispiele

Die folgenden Beispiele sollen den Erfindungsgegenstand näher erläutern:

### Beispiel 1: Herstellung von nanoskaligem Salicylsäure-N-octylamid

0,5 g Salicylsäure-N-octylamid (Smp. ca. 45°C) wurden in 100 g entionisiertem Wasser gelöst und die Mischung auf etwa 50°C erwärmt, wobei sich ein zweiphasiges Gemisch aus Wasser- und Amidphase bildete. Letztere wurde durch Zugabe von 8,9 g Alkylethersulfat (Texapon® N 70, Henkel KGaA, Düsseldorf) unter Ausbildung einer klaren Mischung emulgiert. Der sukzessive Übergang der Ölphase in die transparente Wasser/Amid/Emulgatormischung kann dabei als Indiz für die Ausbildung einer Mikroemulsion angesehen werden. Unter fortwährendem Rühren wurde die Mikroemulsion auf Umgebungstemperatur abgekühlt und anschließend am Rotationsverdampfer bis zur Trockne eingeengt, wobei 9,4 g des in der Ethersulfat-Matrix eingeschlossenen Salicylsäure-N-octylamids in nanopartikulärer Form erhalten wurden. Die Nanopartikel konnten mit der zehnfachen Menge Wasser wieder zu einer stabilen und transparenten Dispersion verarbeitet werden. In der Lichtstreuung zeigten die Partikel bei numerischer Wichtung ein Maximum bei einer Teilchengröße von 120 nm.

### Beispiel 2: Herstellung einer nanoskaligen wässrigen Salicylsäure-N-octylamid-Dispersion

1,0 g Salicylsäure-N-octylamid (Smp. ca. 45°C) wurden mit 30 g entionisiertem Wasser, 30 g Polydiol 400 (PEG-8) und 2 g Polyoxyethylen-glycerinfettsäureester (Tagat S) unter langsamer Erwärmung auf 52°C emulgiert. Anschließend wurden 30 g Fettsäureamidoalkylbetain (Tego Betain BL 215) zugegeben, wobei sich eine klare, stabile Dispersion ausbildete. Anschließend ließ man auf Raumtemperatur abkühlen. Man erhielt 93 g einer transparenten Dispersion. In der Lichtstreuung zeigten die Partikel bei numerischer Wichtung ein Maximum bei einer Teilchengröße von 15 nm.

### Beispiel 3: Rezepturbeispiel für eine deodorierende Pumpzerstäuber-Formulierung:

| Inhaltsstoff | Massengehalt (%) |
|---|---|
| Gehärtetes Rizinusöl mit 40 Mol EO (Eumulgin HRE 40 von Henkel) | 2 |
| Wässrige Dispersion von nanoskaligem Salicylsäure-N-octylamidaus Beispiel 2 | 10 |
| Parfümöl | 0,3 |
| Glycerin | 7,7 |
| Wasser | 80 |

## Patentansprüche

1. Verwendung von nanoskaligen antimikrobieiten Wirkstoffen ausgenommen feinteilige Oxide, Oxidhydrate oder Hydroxide des Calciums, Magnesiums, Aluminiums, Titans, Zirkoniums oder des Zink mit einem Teilchendurchmesser im Bereich von 5 bis 500 nm zur Herstellung von Körperdeodorantien.

2. Verwendung nach Anspruch 1, **dadurch gekennzeichnet, daß** die antimikrobiellen Wirkstoffe gegen grampositive Bakterien wirksam sind.

3. Verwendung nach den Ansprüchen 1 und/oder 2, **dadurch gekennzeichnet, daß** die antimikrobiellen Wirkstoffe gegen Corynebacterium xerosis wirksam sind.

4. Verwendung nach mindestens einem der Ansprüche 1 bis 3, **dadurch gekennzeichnet, daß** man als antimikrobiellen Wirkstoff Salicylsäure-n-octylamid und/oder Salicylsäure-n-decylamid einsetzt.

5. Verwendung nach mindestens einem der Ansprüche 1 bis 3, **dadurch gekennzeichnet, daß** man als antimikrobiellen Wirkstoff 2,4,4'-Tächior-2'-hydroxydiphenylether einsetzt

6. Verwendung nach mindestens einem der Ansprüche 1 bis 3, **dadurch gekennzeichnet, daß** man als antimikrobiellen Wirkstoff einen antimikrobiell wirksamen Riechstoff einsetzt.

7. Verwendung nach mindestens einem der Ansprüche 1 bis 6, **dadurch gekennzeichnet, daß** man nanoskalige Wirkstoffe einsetzt, die man erhält, indem man
(a) Wirkstoffe in eine flüssige Phase, in der sie nicht löslich sind, einbringt,
(b) die resultierende Mischung über den Schmelzpunkt der Wirkstoffe erwärmt,
(c) der resultierenden Ölphase eine wirksame Menge mindestens eines Emulgators oder Schutzkolloids zusetzt und schließlich
(d) die Emulsion unter den Schmelzpunkt der Wirkstoffe abkühlt.

8. Verwendung nach mindestens einem der Ansprüche 1 bis 7, **dadurch gekennzeichnet, daß** man Nanopartikel einsetzt, welche von einem oder mehreren Emulgatoren und/oder Schutzkolloiden ummantelt vorliegen.

9. Verwendung nach mindestens einem der Ansprüche 1 bis 8, **dadurch gekennzeichnet, daß** man die nanoskaligen Wirkstoffe in solchen Mengen einsetzt, daß die Konzentration der in den Nanopartikeln enthaltenen antimikrobiellen Wirkstoffe 0,01 bis 5 Gew.-% bezogen auf die Zubereitungen beträgt.

10. Verwendung nach mindestens einem der Ansprüche 1 bis 9, **dadurch gekennzeichnet, daß** man die nanoskaligen Wirkstoffe zur Herstellung von deodorierenden Aerosolen, Pumpsprays, Roll-ons und Stiften einsetzt.

11. Antimikrobiellen Wirkstoff enthaltendes Körperdeodorans, **dadurch gekennzeichnet, daß** der antimikrobielle Wirkstoff ausgenommen feinteilige Oxide, Oxidhydrate oder Hydroxide des Calciums, Magnesiums, Aluminiums, Titans, Zirkoniums oder des Zink in Form von Nanopartikeln mit einem Teilchendurchmesser im Bereich von 5 bis 500 nm eingearbeitet ist.

## Claims

1. The use of nanoscale antimicrobial active principles, with the exception of finely divided oxides, oxide hydrates or hydroxides of calcium, magnesium, aluminium, titanium, zirconium or zinc, with a particle diameter in the range from 5 to 500 nm for the production of body deodorants.

2. The use claimed in claim 1, **characterized in that** the antimicrobial active principles are active against gram-positive bacteria.

3. The use claimed in claims 1 and/or 2, **characterized in that** the antimicrobial active principles are active against Corynebacterium xerosis.

4. The use claimed in at least one of claims 1 to 3, **characterized in that** salicylic acid-n-octyl amide and/or salicylic acid-n-decyl amide are used as the antimicrobial active principle.

5. The use claimed in at least one of claims 1 to 3, **characterized in that** 2,4,4'-trichloro-2'-hydroxydiphenyl ether is used as the antimicrobial active principle.

6. The use claimed in at least one of claims 1 to 3, **characterized in that** an antimicrobially active aroma substance is used as the antimicrobial active principle.

7. The use claimed in at least one of claims 1 to 6, **characterized in that** nanoscale active principles obtained by
(a) introducing active principles into a liquid phase in which they are insoluble,
(b) heating the resulting mixture to beyond the melting point of the active principles,
(c) adding an effective quantity of at least one emulsifier or protective colloid to the resulting oil phase and finally
(d) cooling the emulsion to below the melting point of the active principles are used.

8. The use claimed in at least one of claims 1 to 7, **characterized in that** nanoparticles coated with one or more emulsifiers and/or protective colloids are used.

9. The use claimed in at least one of claims 1 to 8, **characterized in that** the nanoscale active principles are used in such quantities that the concentration of the antimicrobial active principles present in the nanoparticles is from 0.01 to 5% by weight, based on the preparations.

10. The use claimed in at least one of claims 1 to 9, **characterized in that** the nanoscale active principles are used for the production of deodorizing aerosols, pump sprays, roll-ons and sticks.

11. A body deodorant containing an antimicrobial active principle, **characterized in that** the antimicrobial active principle, with the exception of finely divided oxides, oxide hydrates or hydroxides of calcium, magnesium, aluminium, titanium, zirconium or zinc, is incorporated in the form of nanoparticles with a particle diameter in the range of from 5 to 500 nm.

## Revendications

1. Utilisation de substances actives antimicrobiennes nanométriques, à l'exception des oxydes finement divisés, des hydrates d'oxydes ou des hydroxydes du calcium, du magnésium, de l'aluminium, du titane, du zirconium ou du zinc, présentant un diamètre de particules dans la plage de 5 à 500 nm pour la préparation de déodorants corporels.

2. Utilisation selon la revendication 1, **caractérisée en ce que** les substances actives antimicrobiennes sont actives contre les bactéries Gram-positives.

3. Utilisation selon la revendication 1 et/ou la revendication 2, **caractérisée en ce que** les substances actives antimicrobiennes sont actives contre le Corynebacterium xerosis.

4. Utilisation selon au moins l'une quelconque des revendications 1 à 3, **caractérisée en ce qu'**on utilise comme substance active antimicrobienne le n-octylamide de l'acide salicylique et/ou le n-décylamide de l'acide salicylique.

5. Utilisation selon au moins l'une quelconque des revendications 1 à 3, **caractérisée en ce qu'**on utilise comme substance active antimicrobienne le 2,4,4'-trichloro-2'-hydroxy-diphényléther.

6. Utilisation selon au moins l'une quelconque des revendications 1 à 3, **caractérisée en ce qu'**on utilise comme substance active antimicrobienne une substance odoriférante présentant une activité antimicrobienne.

7. Utilisation selon au moins l'une quelconque des revendications 1 à 6, **caractérisée en ce qu'**on utilise des substances actives nanométriques, qui sont obtenues par
a) l'introduction des substances actives dans une phase liquide dans laquelle elles ne sont pas solubles,
b) le chauffage du mélange obtenu au-dessus du point de fusion des substances actives,
c) l'ajout à la phase huileuse obtenue d'une quantité active d'au moins un émulsifiant ou colloïde de protection et, enfin
d) le refroidissement de l'émulsion en dessous du point de fusion des substances actives.

8. Utilisation selon au moins l'une quelconque des revendications 1 à 7, **caractérisée en ce qu'**on utilise des nanoparticules, qui sont enrobées par un ou plusieurs émulsifiants et/ou colloïdes de protection.

9. Utilisation selon au moins l'une quelconque des revendications 1 à 8, **caractérisée en ce qu'**on utilise les substances actives nanométriques en des quantités telles que la concentration des substances actives antimicrobiennes contenues dans les nanoparticules est de 0,01 à 5% en poids par rapport aux compositions.

10. Utilisation selon au moins l'une quelconque des revendications 1 à 9, **caractérisée en ce qu'**on utilise les substances actives nanométriques pour la production d'aérosols de déodorant, de sprays à pompe de déodorant, de déodorants à bille et de déodorants en stick.

11. Déodorant corporel contenant une substance active antimicrobienne, **caractérisé en ce que** la substance active antimicrobienne, à l'exception des oxydes finement divisés, des hydrates d'oxydes ou des hydroxydes du calcium, du magnésium, de l'aluminium, du titane, du zirconium ou du zinc, est incorporée sous forme de nanoparticules présentant un diamètre des particules dans la plage de 5 à 500 nm.
